# EUROPEAN PATENT SPECIFICATION

(11) **EP 1 868 452 B1**
(45) Date of publication and mention of the grant of the patent: **22.07.2009**
(21) Application number: 06724999.5
(22) Date of filing: 09.03.2006
(51) Int. Cl.: A23L 1/226, A61K 47/10, C07C 49/825, C07C 49/84

(54) **HYDROXYDEOXYBENZOINS AND THE USE THEREOF TO MASK A BITTER TASTE**
HYDROXYDESOXYBENZOINE UND IHRE VERWENDUNG ZUR MASKIERUNG VON BITTERGESCHMACK
HYDROXYDEOXYBENZOINES PERMETTANT DE MASQUER UN GOUT AMER

(30) Priority: 04.04.2005 US 668235 P
(43) Date of publication of application: 26.12.2007
(73) Proprietor: Symrise GmbH & Co. KG, 37603 Holzminden (DE)
(72) Inventor: LEY, Jakob, 37603 Holzminden (DE); KINDEL, Günter, 37671 Höxter (DE); PAETZ, Susanne, 37671 Höxter (DE); KRAMMER, Gerhard, 37603 Holzminden (DE)
(74) Representative: Stilkenböhmer, Uwe Michael
(86) International application number: PCT/EP2006/060591
(87) International publication number: WO 2006/106023

(56) References cited:
- WO-A-20/06024587
- DE-A1- 10 160 721
- WAHALA K ET AL: "EXPEDIENT SYNTHESIS OF POLYHYDROXYISOFLAVONES" JOURNAL OF THE CHEMICAL SOCIETY, PERKIN TRANSACTIONS 1, CHEMICAL SOCIETY. LETCHWORTH, GB, 1991, pages 3005-3008, XP009017947 ISSN: 0300-922X cited in the application
- DUBOIS G E ET AL: "Dihydrochalcone Sweeteners. A study of the Atypical Temporal Phenomena" JOURNAL OF MEDICINAL CHEMISTRY, AMERICAN CHEMICAL SOCIETY. WASHINGTON, US, vol. 24, 1981, pages 408-428, XP002345673 ISSN: 0022-2623 cited in the application
- MAKOTO T. ET AL.: "Chemical Studies on the Oriental Plant Drugs" CHEM. PHARM. BULL., vol. 20, no. 11, 1972, pages 2488-2490, XP009068550

## Description

The invention relates to the use of hydroxydeoxybenzoins (i.e. hydroxy-substituted 1,2-diphenylethanones), salts thereof and mixtures thereof to mask or reduce unpleasant taste impressions, in particular bitter, astringent and/or metallic taste impressions. The invention relates also to specific preparations comprising an effective amount of the designated hydroxydeoxybenzoins, salts thereof or mixtures thereof.

Foodstuffs or snacks frequently comprise various bitter substances which on the one hand are desirable and characteristic in moderation (e.g. caffeine in tea or coffee, quinine in so-called bitter lemon drinks, hop extracts in beer) but on the other hand can also reduce the value considerably (e.g. flavonoid glycosides and limonoids in citrus juices, bitter after-taste of many artificial sweeteners such as aspartame or saccharin, hydrophobic amino acids and/or peptides in cheese).

Subsequent treatment is therefore often necessary to reduce the natural content of bitter substances, for example treatment by extraction, as in the decaffeination of tea or coffee, or treatment with enzymes, for example treatment of orange juice with a glycosidase in order to destroy the bitter naringin, or the use of specific peptidases in the ripening of cheese. Such treatment puts a strain on the product, produces waste materials and, for example, is also the cause of solvent residues and other residues (enzymes) in the products.

It is therefore desirable to find substances which are able to effectively suppress, or at least reduce, unpleasant taste impressions, in particular bitter, astringent and/or metallic taste impressions.

It is particularly important to suppress the bitter taste in many pharmaceutical active ingredients, because the willingness of the patient, in particular of patients who are sensitive to bitterness, such as children, to take the preparation orally can be markedly increased as a result. Many pharmaceutical active ingredients, for example aspirin, salicin, paracetamol, ambroxol or quinine, to name but only a few for the purposes of illustration, have a pronounced bitter, astringent and/or metallic taste and/or after-taste.

Although some substances are known that are able partly to suppress the bitter taste, many of them exhibit considerable limitations in use.

In US 5,637,618, a bitter taste is reduced by means of lactisol (2O-(4-methoxyphenyl)lactic acid]. However, this inhibitor at the same time exhibits pronounced inhibition of the sweet taste impression (see US 5,045,336), which limits its usability considerably.

2,4-Dihydroxybenzoic acid potassium salt is described in US 5,643,941 (table, column 3, line 18) as an agent for masking the bitter taste of potassium chloride, but it is not able to suppress the taste of caffeine, for example.

According to GB 2,380,936, the taste of bitter pharmaceuticals is suppressed using ginger extracts. However, the strong flavour impression and/or the sharpness, frequently to be found therein, of ginger extracts or active constituents thereof is not suitable for a large number of applications.

Neohesperidine dihydrochalcone also exhibits a bitter-reducing effect, but is primarily a sweetener (see Manufacturing Chemist 2000, July edition, p. 16-17) which also has a disturbing effect in non-sweet applications.

Although US-A 5,580,545 describes taste-altering properties for some flavones (2-phenylchrom-2-en-4-ones), a bitter-reducing or -suppressing action has not been found.

US 2002 177,576 describes the suppression of a bitter taste using nucleotides, for example cytidine-5'-monophosphate (CMP). However, the compounds, which are strongly polar and can therefore be used only in strongly polar solvents, can be used to only a very limited extent in many fat-containing foodstuffs. In addition, the availability of such substances is greatly limited owing to their complex chemical synthesis.

In US 2002 188,019, hydroxyflavanones are described as effective bitter-masking agents, but they are obtainable synthetically only with difficulty and are not available inexpensively in larger amounts.

The sodium salts sodium chloride, sodium citrate, sodium acetate and sodium lactate exhibit a bitter-masking effect against many bitter substances (e.g. Nature, 1997, Vol. 387, p. 563); however, the ingestion of relatively large amounts of sodium ions can lead, for example, to cardiocirculatory diseases as a result of raised blood pressure. In addition, it is disadvantageous that a significant bitter-masking effect only occurs at relatively high sodium concentrations (above about 0.1 M), which corresponds, for example, to a generally unacceptably large amount of about 0.6 wt.% NaCl and accordingly a pronounced salty taste in the final application (see R.S.J. Keast, P.A.S. Breslin and G.K. Beauchamp, Chimia 2001, 55(5), 441-447).

In WO 00/21390, polyglutamic acid is described as a bitterness-masking agent; relatively high concentrations in the region of about 1 wt.% are required.

A lipoprotein consisting of β-lactoglobulin and phosphatidic acid also exhibits a bitter-masking effect (EP-A 635 218). Such polymers are difficult to characterise and standardise, however, and exhibit a markedly soapy after-taste.

The flavone glycoside neodiosmin [5,7-dihydroxy-2-(4-methoxy-3-hydroxyphenyl)7-O-neohesperidosyl-chrom-2-en-4-one] likewise exhibits a bitter-masking action (US-A 4,154,862) but is distinguished by a disaccharide residue, which makes the substance much more difficult to prepare, or isolate, and use.

The primary object of the present invention was to find substances which are suitable (a) for masking or reducing the unpleasant taste impression of substances that taste unpleasant (and preferably in particular exhibit a bitter-masking effect against a large number of bitter substances) and/or (b) for enhancing the sweet taste of a substance that tastes sweet, (c) are widely usable and (d) are readily accessible.

The stated object is achieved according to the invention by using hydroxydeoxybenzoins of formula (I) 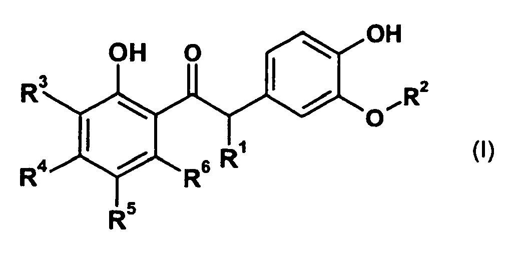 wherein
R¹ and R² independently of one another represent hydrogen or lower alkyl,
R³, R⁴, R⁵ and R⁶ independently of one another represent hydrogen, hydroxy or lower alkoxy,
salts thereof and mixtures thereof to mask or reduce the unpleasant taste impression of a substance that tastes unpleasant, that is to say as a taste-correcting agent, and/or to enhance the sweet taste of a substance that tastes sweet.

Lower alkyl radicals within the scope of the invention are branched, cyclic or linear C₁ to C₅ alkyl chains, with preference being given to: methyl, ethyl, 1-propyl, 2-propyl.

Lower alkoxy radicals within the scope of the invention are branched, cyclic or linear C₁ to C₅ alkoxy chains, with preference being given to: methoxy, ethoxy, 1-propoxy, 2-propoxy.

Substances that taste unpleasant within the scope of the invention are:
(a) substances that taste bitter, astringent, cardboardy, chalky, dusty, dry, floury, rancid and/or metallic and
(b) substances that have a bitter, astringent, cardboardy, chalky, dusty, dry, floury, rancid or metallic after-taste.

The above-mentioned substances that taste unpleasant may also possess further taste and/or odour qualities which are generally not unpleasant. As further not unpleasant taste qualities within the scope of the present invention there may be mentioned, for example, the impressions spicy, umami, sweet, salty, sour, sharp, cooling, warming, burning or tingling.

Substances that taste bitter, astringent, cardboardy, chalky, dusty, dry, floury, rancid or metallic are, for example: xanthine alkaloids, xanthines (caffeine, theobromine, theophylline), alkaloids (quinine, brucine, strychnine, nicotine), phenolic glycosides (e.g. salicin, arbutin), flavonoid glycosides (e.g. hesperidine, naringin), chalcones or chalcone glycosides, hydrolysable tannins (gallic or ellagic acid esters of carbohydrates, e.g. pentagalloylglucose), non-hydrolysable tannins (optionally galloylated catechols or epicatechols and oligomers thereof, e.g. proanthyocyanidines or procyanidines, thearubigin), flavones (e.g. quercertin, taxifolin, myricetin), other polyphenols (γ-oryzanol, coffeic acid or esters thereof), terpenoid bitter substances (e.g. limonoids such as limonine or nomilin from citrus fruits, lupolones and humulones from hops, iridoids, secoiridoids), absinthin from wormwood, amarogentin from gentian, metal salts (potassium chloride, sodium sulfate, magnesium salts, iron salts, aluminium salts, zinc salts), pharmaceutical active ingredients (e.g. fluoroquinolone antibiotics, paracetamol, aspirin, β-lactam antibiotics, ambroxol, propylthiouracil [PROP], guaifenesin), vitamins (for example vitamin H, vitamins from the B group, such as vitamin B1, B2, B6, B12, niacin, pantothenic acid), denatonium benzoate or other denatonium salts, sucralose octaacetate, urea, unsaturated fatty acids, in particular unsaturated fatty acids in emulsions, amino acids (e.g. leucine, isoleucine, valine, tryptophan, proline, histidine, tyrosine, lysine or phenylalanine), peptides (in particular peptides having an amino acid from the group leucine, isoleucine, valine, tryptophan, proline or phenylalanine at the N- or C-terminus).

Substances that have a bitter, astringent, cardboardy, chalky, dusty, dry, floury, rancid or metallic after-taste can be flavourings or taste-imparting substances having a not unpleasant primary taste (for example sweet, salty, spicy, sour) and/or odour and can belong, for example, to the group of the sweeteners, sugar substitutes or flavourings. Examples which may be mentioned include: aspartame, neotame, superaspartame, saccharin, sucralose, tagatose, monellin, stevioside, thaumatin, miraculin, glycyrrhizin, glycyrrhetinic acid or derivatives thereof, cyclamates or the pharmaceutically acceptable salts of the above-mentioned compounds.

Substances (including plant extracts) that have a sweet taste can be, for example, sweet-tasting carbohydrates (e.g. sucrose, trehalose, lactose, maltose, melizitose, raffinose, palatinose, lactulose, D-fructose, D-glucose, D-galactose, L-rhamnose, D-sorbose, D-mannose, D-tagatose, D-arabinose, L-arabinose, D-ribose, D-glyceraldehyde), sugar alcohols (e.g. erythritol, threitol, arabitol, ribitol, xylitol, sorbitol, mannitol, dulcitol, lactitol), proteins (e.g. miraculin, monellin, thaumatin, curculin, brazzein), sweeteners (magap, sodium cyclamate, acesulfame K, neohesperidine dihydrochalcone, saccharin sodium salt, aspartame, superaspartame, neotame, sucralose, stevioside, rebaudioside, lugduname, carrelame, sucrononate, sucrooctate), specific sweet-tasting amino acids (glycine, D-leucine, D-threonine, D-asparagine, D-phenylalanine, D-tryptophan, L-proline), other sweet-tasting low molecular weight substances (e.g. hernandulcin, dihydrochalcone glycosides, glycyrrhetinic acid derivatives), extracts of liquorice *(Glycyrrhizza glabra spp.),* sugar beet *(Beta vulgaris spp.),* sugar cane *(Saccharum officinarum spp.)* or *Stevia spp.* (e.g. *Stevia rebaudiana).*

Preference is given to the use of hydroxydeoxybenzoins of formula (I) above wherein
- R¹: represents hydrogen,
- R²: represents hydrogen or a lower alkyl radical,
- R³ and R⁵: represent hydrogen
and
at least one of the radicals R⁴ and R⁶ represents hydroxy or lower alkoxy,
salts thereof and mixtures thereof.

Particular preference is given to the use of hydroxydeoxybenzoins of formula (I) above wherein
- R¹: represents hydrogen,
- R²: represents hydrogen or methyl,
- R³ and R⁵: represent hydrogen
and
at least one of the radicals R⁴ and R⁶ repre sents hydroxy or methoxy and the other preferably represents hydrogen or hydroxy or methoxy,
salts thereof and mixtures thereof.

Particular preference is given to the use of
2-(4-hydroxy-3-methoxyphenyl)-1-(2,4,6-trihydroxyphenyl)ethanone (compound 1)
1-(2,4-dihydroxyphenyly2-(4-hydroxy-3-methoxyphenyl)ethanone (compound 2)
1-(2-hydroxy-4-methoxyphenyl)-2-(4-hydroxy-3-methoxyphenyl)ethanone (compound 3),
salts thereof and mixtures thereof.

The structures of the preferred compounds (1) to (3) are show hereinbelow for the purposes of clarification.

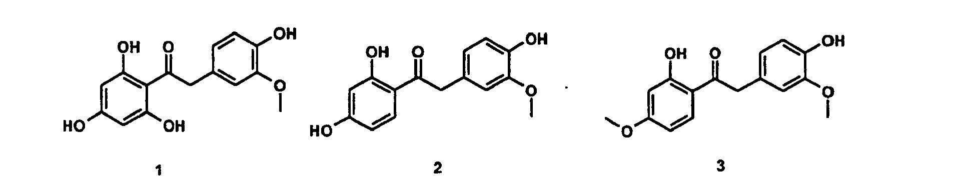

Of course, the various hydroxydeoxybenzoins that are to be used according to the invention and the salts thereof can in each case be employed in accordance with the invention alone or in the form of mixtures.

In salts of a hydroxydeoxybenzoin of formula (I) above that are to be used according to the invention (wherein the comments made above in respect of the preferred meanings of the radicals apply), one, more than one or all of the hydroxy groups of the hydroxydeoxybenzoin have been deprotonated. A corresponding amount of counter-cations are then present, which counter-cations are preferably selected from the group consisting of: singly positively charged cations of the first main and subsidiary group, ammonium ions, trialkylammonium ions, doubly positively charged cations of the second main and subsidiary group, and triply positively charged cations of the third main and subsidiary group, and mixtures thereof. It will be understood that the number of hydroxy groups in the underlying hydroxydeoxybenzoin determines the maximum degree of deprotonation and accordingly also the amount of counter-cations present. If, for example, a total of two hydroxy groups are present in the underlying hydroxydeoxybenzoin, then a doubly negatively charged anion is present on complete deprotonation of the hydroxy groups, so that a corresponding number of positive charges must be provided by the counter-cation(s).

Particularly preferred cations are Na⁺, K⁺, NH₄⁺, Ca²⁺, Mg²⁺, Al³⁺ and Zn²⁺.

The synthesis of compound 1 is described in DE 10160721, for example.

Specific compounds of formula 1 are described, for example, as synthesis intermediates for the preparation of isoflavones (see K. Wähälä and T.A. Hase in: J. Chem. Soc. Perkin Trans. 1, 1991, pages 3005-3008).

In J. Med. Chem., 1981, Volume 24, No. 4, pages 408-428, DuBois *et al.* describe a structurally isomeric compound 2-(3-hydroxy-4-methoxyphenyl)-1-(2,4,6-trihydroxyphenyl)ethanone (compound no. 16 therein) which was studied within the context of various sweeteners but was found to be tasteless. There is no indication in the mentioned publication that the described compound or the position isomers thereof might have taste-modulating, in particular masking actions against unpleasant taste impressions. The important factor in this study was the existence of a 3-hydroxy-4-methoxyphenyl group, which is not suitable for use within the scope of the present invention.

The use of the hydroxydeoxybenzoins to be used according to the invention as flavouring or taste-imparting substances or to influence flavour or taste has not been described or rendered obvious in any of the mentioned cases.

Surprisingly, it has been found that the hydroxydeoxybenzoins used according to the invention are able, even in very low concentrations, to reduce or even suppress completely the unpleasant taste impression, in particular the bitter taste impression, of a large number of substances, in particular of methylxanthines, such as, for example, caffeine, alkaloids, such as, for example, quinine, flavonoids, such as, for example, naringin, phenols, such as, for example, salicin, inorganic salts, such as potassium chloride or magnesium sulfate, pharmaceutical active ingredients, such as, for example, denatonium benzoate or β-lactam antibiotics, it being particularly advantageous that the hydroxydeoxybenzoins used according to the invention possess virtually no taste of their own and are able to enhance the further, generally not unpleasant taste qualities, in particular the sweet taste quality.

As already mentioned, one aspect of the present invention relates to the use of a hydroxydeoxybenzoin, salt or mixture according to the invention to mask or reduce the unpleasant taste impression of a substance that tastes unpleasant or to enhance not unpleasant taste impressions, that is to say as a taste-correcting agent. The hydroxydeoxybenzoin, the salt or the mixture is preferably used in a preparation for nutrition, oral care or enjoyment or in an oral pharmaceutical preparation or in a cosmetic preparation for application in the region of the head, the preparation conventionally comprising one or more substances that taste unpleasant.

A further aspect of the present invention relates to such preparations. Preparations according to the invention for nutrition, oral care or enjoyment or cosmetic preparations according to the invention for application in the region of the head preferably comprise from 0.000001 wt.% to 95 wt.%, based on the total weight of the preparation, of a hydroxydeoxybenzoin, salt or mixture according to the invention.

An oral pharmaceutical preparation according to the invention preferably comprises from 0.0001 wt.% to 10 wt.%, based on the total weight of the preparation, of a hydroxydeoxybenzoin, salt or mixture according to the invention.

Of particular relevance are preparations according to the invention comprising at least one substance that tastes unpleasant, wherein the amount of the substance that tastes unpleasant is sufficient to be perceived as an unpleasant taste in a comparative preparation that does not comprise any hydroxydeoxybenzoin, salt or mixture according to the invention but otherwise has an identical composition, and the amount of the hydroxydeoxybenzoin, salt or mixture according to the invention in the preparation is sufficient to mask sensorially the unpleasant taste impression of the substance that tastes unpleasant or to reduce it in comparison with the comparative preparation.

Preparations according to the invention can be in the form of semi-finished products, in the form of fragrance, flavouring or taste-imparting compositions or in the form of a spice mixture.

Preparations for nutrition or enjoyment within the scope of the invention are, for example, baked goods (e.g. bread, dry biscuits, cakes, other baked goods), confectionery (e.g. chocolates, chocolate bars, other products in bar form, fruit gums, hard and soft caramels, chewing gum), alcoholic or non-alcoholic drinks (e.g. coffee, tea, wine, drinks containing wine, beer, drinks containing beer, liqueurs, whiskies, brandies, soft drinks containing fruit, isotonic drinks, refreshment drinks, nectars, fruit and vegetable juices, fruit or vegetable juice preparations), instant drinks (e.g. instant cocoa drinks, instant tea drinks, instant coffee drinks), meat products (e.g. ham, fresh sausage or raw sausage preparations, spiced or marinated fresh or salt meat products), eggs or egg products (dried egg, egg white, egg yolk), cereal products (e.g. breakfast cereals, muesli bars, pre-cooked finished rice products), milk products (e.g. milk drinks, milk ice, yoghurt, kefir, fresh cheese, soft cheese, hard cheese, dried milk powder, whey, butter, buttermilk, partially or fully hydrolysed milk-protein-containing products), products made from soya protein or other soybean fractions (e.g. soya milk and products produced therefrom, soya-lecithin-containing preparations, fermented products such as tofu or tempe or products made therefrom, soya sauces), fruit preparations (e.g. jams, fruit ice, fruit sauces, fruit fillings), vegetable preparations (e.g. ketchup, sauces, dried vegetables, frozen vegetables, pre-cooked vegetables, vegetables pickled in vinegar, cooked vegetables), snack articles (e.g. baked or fried potato crisps or potato pulp products, bread dough products, corn- or peanut-based extrudates), products based on fat and oil or emulsions thereof (e.g. mayonnaise, remoulade, dressings, spice preparations), other ready meals and soups (e.g. dried soups, instant soups, pre-cooked soups), spices, spice mixtures and also, especially, seasonings, which are used, for example, in the snacks sector. The preparations within the scope of the invention may also be used as semi-finished products for the production of further preparations for nutrition or enjoyment. The preparations within the scope of the invention may also be in the form of capsules, tablets (uncoated and coated tablets, e.g. enteric coatings), dragées, granules, pellets, solids mixtures, dispersions in liquid phases, in the form of emulsions, in the form of powders, in the form of solutions, in the form of pastes or in the form of other swallowable or chewable preparations as food supplements.

Preparations for oral care within the scope of the invention are in particular oral and/or tooth care agents, such as toothpastes, tooth gels, tooth powders, mouthwashes, chewing gums and other oral care agents.

Oral pharmaceutical preparations within the scope of the invention are preparations which are in the form of, for example, capsules, tablets (uncoated and coated tablets, e.g. enteric coatings), dragées, granules, pellets, solids mixtures, dispersions in liquid phases, in the form of emulsions, in the form of powders, in the form of solutions, in the form of pastes or in the form of other swallowable or chewable preparations and which are used as medicaments available only on prescription, medicaments available only at a pharmacy or other medicaments or as food supplements.

Cosmetic preparations for application in the region of the head are in particular those which contain a substance that tastes unpleasant and which may come into contact with the oral cavity even when applied properly to the skin, that is to say, for example, - as already mentioned - cosmetic preparations for application in the region of the head, such as soaps, other cleansing or care agents for the facial region, face creams or lotions or ointments, sun protection agents, beard cleansing or care agents, shaving foams, soaps or gels, lipsticks or other cosmetics for the lips, or lip care agents.

Further conventional active ingredients, basic substances, auxiliary substances and additives for preparations for nutrition, oral care or enjoyment or for oral pharmaceutical preparations or for cosmetic preparations for application in the region of the head may be present in amounts of from 5 to 99.999999 wt.%, preferably from 10 to 80 wt.%, based on the total weight of the preparation. The preparations may further comprise water in an amount of up to 99.999999 wt.%, preferably from 5 to 80 wt.%, based on the total weight of the preparation.

According to a preferred embodiment, the preparations according to the invention comprising one or more of the hydroxydeoxybenzoins according to the invention, or salts or mixtures thereof, are prepared by incorporating the hydroxydeoxybenzoins according to the invention, or salts or mixtures thereof, in the form of substances, in the form of a solution or in the form of a mixture with a solid or liquid carrier into a base preparation for nutrition, oral care or enjoyment or into an oral pharmaceutical base preparation. Advantageously, preparations according to the invention in the form of a solution can also be converted into a solid preparation by spray-drying.

According to a further preferred embodiment, for the production of preparations according to the invention, the hydroxydeoxybenzoins or salts or mixtures thereof that are to be used according to the invention, and optionally other constituents of the preparation according to the invention, are incorporated beforehand into emulsions, into liposomes, e.g. starting from phosphatidyl choline, into microspheres, into nanospheres or into capsules, granules or extrudates of a matrix suitable for foodstuffs and snacks, e.g. a matrix of starch, starch derivatives, cellulose or cellulose derivatives (e.g. hydroxypropylcellulose), other polysaccharides (e.g. alginate), natural fats, natural waxes (e.g. beeswax, carnauba wax) or of proteins, e.g. gelatin.

In a further preferred preparation process, the hydroxydeoxybenzoins or salts or mixtures thereof are complexed beforehand with one or more suitable complexing agents, for example with cyclodextrins or cyclodextrin derivatives, preferably α- or β-cyclodextrin, and used in that complexed form.

Particular preference is given to a preparation according to the invention in which the matrix is so chosen that the hydroxydeoxybenzoins are released from the matrix in a delayed manner so that a long-lasting action is obtained.

As further constituents of preparations according to the invention for nutrition or enjoyment there may be used basic substances, auxiliary substances and additives conventional for foodstuffs or snacks, for example water, mixtures of fresh or processed, vegetable or animal base or raw substances (e.g. raw, roast, dried, fermented, smoked and/or boiled meat, bone, cartilage, fish, vegetables, fruits, herbs, nuts, vegetable or fruit juices or pastes or mixtures thereof), digestible or non-digestible carbohydrates (e.g. sucrose, maltose, fructose, glucose, dextrins, amylose, amylopectin, inulin, xylans, cellulose, tagatose), sugar alcohols (e.g. sorbitol, erythritol), natural or hardened fats (e.g. tallow, lard, palm oil, coconut fat, hardened vegetable fat), oils (e.g. sunflower oil, groundnut oil, maize oil, olive oil, fish oil, soybean oil, sesame oil), fatty acids or their salts (e.g. potassium stearate), proteinogenic or non-proteinogenic amino acids and related compounds (e.g. γ-aminobutyric acid, taurine), peptides (e.g. glutathione), natural or processed proteins (e.g. gelatin), enzymes (e.g. peptidases), nucleic acids, nucleotides, other taste-correcting agents for unpleasant taste impressions, taste modulators for further, generally not unpleasant taste impressions, taste-modulating substances (e.g. inositol phosphate, nucleotides such as guanosine monophosphate, adenosine monophosphate or other substances such as sodium glutamate or 2-phenoxypropionic acid), emulsifiers (e.g. lecithins, diacylglycerols, gum arabic), stabilisers (e.g. carrageenan, alginate), preservatives (e.g. benzoic acid, sorbic acid), antioxidants (e.g. tocopherol, ascorbic acid), chelators (e.g. citric acid), organic or inorganic acidifying agents (e.g. malic acid, acetic acid, citric acid, tartaric acid, phosphoric acid), additional bitter substances (e.g. quinine, caffeine, limonine, amarogentin, humulones, lupolones, catechols, tannins), sweeteners (e.g. saccharin, cyclamate, aspartame, neotame), mineral salts (e.g. sodium chloride, potassium chloride, magnesium chloride, sodium phosphates), substances that prevent enzymatic browning (e.g. sulfite, ascorbic acid), essential oils, plant extracts, natural or synthetic colourings or colouring pigments (e.g. carotinoids, flavonoids, anthocyanins, chlorophyll and derivatives thereof), spices, substances having trigeminal action or plant extracts containing such substances having trigeminal action, synthetic, natural or nature identical flavourings or fragrances and also odour-correcting agents.

Tooth care agents (as the basis for preparations for oral care) containing the hydroxydeoxybenzoins according to the invention, salts or mixtures thereof, generally comprise an abrasive system (abrasive or polishing agent), such as, for example, silicas, calcium carbonates, calcium phosphates, aluminium oxides and/or hydroxyl apatites, surface-active substances, such as, for example, sodium lauryl sulfate, sodium lauryl sarcosinate and/or cocamidopropylbetain, humectants, such as, for example, glycerol and/or sorbitol, thickeners, such as, for example, carboxymethylcellulose, polyethylene glycols, carrageenan and/or Laponite^{®}, sweeteners, such as, for example, saccharin, taste-correcting agents for unpleasant taste impressions, taste-correcting agents for further, generally not unpleasant taste impressions, taste-modulating substances (e.g. inositol phosphate, nucleotides such as guanosine monophosphate, adenosine monophosphate or other substances such as sodium glutamate or 2-phenoxypropionic acid), cooling active ingredients, such as, for example, menthol, menthol derivatives (e.g. L-menthol, L-menthyl lactate, L-menthyl alkylcarbonates, menthone ketals, menthanecarboxylic acid amides), 2,2,2-trialkylacetic acid amides (e.g. 2,2-diisopropylpropionic acid methylamide), icilin derivatives, stabilisers and active ingredients, such as, for example, sodium fluoride, sodium monofluorophosphate, tin difluoride, quaternary ammonium fluorides, zinc citrate, zinc sulfate, tin pyrophosphate, tin dichloride, mixtures of various pyrophosphates, triclosan, cetylpyridinium chloride, aluminium lactate, potassium citrate, potassium nitrate, potassium chloride, strontium chloride, hydrogen peroxide, flavourings and/or sodium bicarbonate or odour-correcting agents.

Chewing gums (as a further example of preparations for oral care) which contain hydroxydeoxybenzoins according to the invention, salts or mixtures thereof, generally comprise a chewing gum base, that is to say a chewable mass which becomes plastic when chewed, sugars of various types, sugar substitutes, sweeteners, sugar alcohols, other taste-correcting agents for unpleasant taste impressions, taste modulators for further, generally not unpleasant taste impressions, taste-modulating substances (e.g. inositol phosphate, nucleotides such as guanosine monophosphate, adenosine monophosphate or other substances such as sodium glutamate or 2-phenoxypropionic acid), humectants, thickeners, emulsifiers, flavourings and stabilisers or odour-correcting agents.

As constituents for oral pharmaceutical preparations according to the invention there may be used any further active ingredients, basic substances, auxiliary substances and additives conventional for oral pharmaceutical preparations. As active ingredients there can be used in particular also orally formulatable pharmaceutical active ingredients that have an unpleasant taste. The active ingredients, basic substances, auxiliary substances and additives can be converted into the oral forms of administration in a manner known *per se.* This is generally effected using inert, non-toxic, pharmaceutically suitable auxiliary substances. These include *inter alia* carriers (e.g. microcrystalline cellulose), solvents (e.g. liquid polyethylene glycols), emulsifiers (e.g. sodium dodecylsulfate), dispersing agents (e.g. polyvinylpyrrolidone), synthetic and natural biopolymers (e.g. albumin), stabilisers (e.g. antioxidants such as ascorbic acid), colourings (e.g. inorganic pigments such as iron oxides) and odour-correcting agents as well as taste-correcting agents that do not affect the bitter taste.

The preparations according to the invention can preferably also comprise a flavouring composition in order to complete and refine the taste and/or odour of the preparation. Suitable flavouring compositions contain, for example, synthetic, natural or nature identical flavourings, fragrances and taste-imparting substances as well as suitable auxiliary substances and carriers. It is considered to be particularly advantageous that a bitter or metallic taste impression coming from flavourings or fragrances contained in the preparations according to the invention can be masked or reduced, and the overall flavour or taste profile can accordingly be improved.

Preparations according to the invention in the form of semi-finished products can be used to mask or reduce the unpleasant taste impression of finished-product preparations which are produced using the semi-finished-product preparation.

Preparations according to the invention which are used as semi-finished products generally comprise from 0.0001 wt.% to 95 wt.%, preferably from 0.001 to 80 wt.%, but especially from 0.01 wt.% to 50 wt.%, based on the total weight of the preparation, of hydroxydeoxybenzoins, salts thereof or mixtures thereof to be used according to the invention.

Preparations to be used according to the invention that are in the form of semi-finished products can be used to mask or reduce the unpleasant taste impression of finished-product preparations which are produced using the semi-finished-product preparation.

In a particularly preferred embodiment of the invention, the hydroxydeoxybenzoins to be used according to the invention, salts or mixtures thereof are used in the preparations according to the invention in combination with at least one further substance for changing, masking or reducing the unpleasant taste impression of a substance that tastes unpleasant. Particularly effective masking can be achieved in this manner. Particular preference is given to the combination of the hydroxydeoxybenzoins to be used according to the invention with other taste-correcting agents for unpleasant, in particular bitter, taste impressions.

The further taste-correcting agents can be selected from the following list without thereby limiting the invention: nucleotides (e.g. adenosine-5'-monophosphate, cytidine-5'-monophosphate) or pharmaceutically acceptable salts thereof, lactisols, sodium salts (e.g. sodium chloride, sodium lactate, sodium citrate, sodium acetate, sodium gluconoate), hydroxyflavanones (e.g. eriodictyol, homoeriodictyol or sodium salts thereof), in particular according to EP 1258200, hydroxybenzoic acid amides (e.g. 2,4-dihydroxybenzoic acid vanillylamide, 4-hydroxybenzoic acid vanillylamide), amino acids or mixtures of whey proteins with lecithins.

### Examples

The Examples serve only to illustrate the invention without limiting it.

### Example 1:2-(4-Hydroxy-3-methoxyphenyl)-1-(2,4,6-trihydroxyphenyl)-ethanone (compound 1)

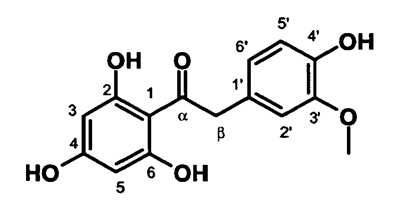

4-Hydroxy-3-methoxybenzylnitrile (1.03 g) was placed together with 1,3,5-trihydroxybenzene (1.43 g) in 20 ml of 1,4-dioxane, and dry hydrogen chloride gas was passed through for 6 hours. The mixture was then stirred for 36 hours at room temperature, and the precipitate was filtered off with suction. The crystallisate was diluted with ice-water (12 ml) and heated to boiling heat for 2 hours. After cooling, the resulting product is isolated, washed with water and dried. Yield 1.13 g (77 % of theory).

HRMS: calc. for C₁₄H₁₅O₆ 290.0790, found 290.0809.

HPLC-MS (RP-18-Phase, APCl+): *m*/*z* = 287.12 (100 %), 288.15 (15.8 %), 290.96 (2.9 %, [M+H]⁺).

¹H-NMR (400 MHz, CD₃OD, internal standard TMS): δ = 6.84 (1H, d, *J* = 1.9 Hz, H-2'), 6.71 (1H, dd *J* = 8.0 Hz, *J* = 0.4 Hz, H-5'), 6.68 (1H, ddd, *J* = 8.0 Hz, *J* = 1.8 Hz, *J* = 0.5 Hz, H-6'), 5.82 (2H, s, H-3 and H-5), 4.29 (1 H, t, *J* = 0.5 Hz, H-β), 3.81 (3H, s, OCH₃) ppm.

¹³C-NMR (100 MHz; CD₃OD, internal standard TMS): δ = 204.99 (C, C-α), 166.39 (C, C-4), 165.92 (C, C-2 and C-6), 148.72 (C, C-3'), 146.13 (C, C-4'), 128.81 (C, C-1'), 123.34 (CH, C-6'), 115.95 (CH, C-5'), 114.54 (CH, C-2'), 105.27 (C, C-1), 95.83 (CH, C-3 and C-5), 55.48 (CH₃, OCH₃), 49.99 (CH₂, C-β) ppm.

### Example 2:1-(2,4-Dihydroxyphenyl)-2-(4-hydroxy-3-methoxyphenyl)-ethanone (compound 2)

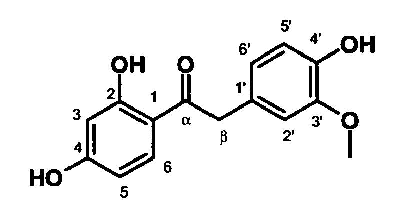

Resorcinol (1.11 g, 10 mmol.), 4-hydroxy-3-methoxyphenylacetic acid (1.82 g, 10 mmol.) and boron trifluoride-ether complex (25 ml, 0.2 mol.) were stirred under a nitrogen atmosphere for 3 hours at 20-25°C and then for 2.5 hours at 55-65°C. A sodium acetate solution (12 g/100 ml water) was added in portions to the cloudy mixture (caution: violent reaction). The reaction mixture was extracted with diethyl ether and the organic phase was dried over Na₂SO₄; filtration was carried out, and the organic phase was concentrated to dryness by evaporation *in vacuo.* The solid residue (4.60 g) was stirred with hydrochloric acid (15 %, 50 ml) to complete the reaction, and the precipitate was filtered off, washed with water and dried.

HPLC-MS (RP-18-Phase, APCl-): *m*/*z* = 269.31 (100 %), 273.29 (32 %, [M-H]⁻), 547.72 (23 %, [2M-H]-), 546.68 (93 %).

¹H-NMR (400 MHz, d₆-DMSO, internal standard TMS): δ = 10.63 (1H, s, OH)) 7.92 (1H, d, *J* = 8.9 Hz, H-5'), 6.41 (1H, d, *J* = 1.9 Hz, H-3), 6.74 (1H, d, *J* = 8.0 Hz, H-6), 6.38 (1 H, dd, *J* = 8.1 Hz, *J* = 1.9 Hz, H-5), 6.36 (1H, dd, *J* = 8.9 Hz, *J* = 2.3 Hz, H-6'), 6.22 (1H, d, *J* = 2.4 Hz, H-2'), 4.12 (2H, s, H-β), 3.71 (3H, s, -OCH₃) ppm.

¹³C-NMR (100 MHz; CD₃OD, internal standard TMS): δ = 202.47 (C, C-α), 164.78 (C, C-4), 164.60 (C, C-2), 147.34 (C, C-3'), 145.19 (C, C-4'), 133.49 (CH, C-6), 125.63 (C, C-1'), 121.62 (CH, C-6'), 115.28 (CH, C-5'), 113.55 (CH, C-2'), 111.99 (C, C-1), 108.09 (CH, C-5), 102.34 (CH, C-3), 55.48 (CH₃, OCH₃), 43.56 (CH₂, C-β) ppm.

### Example 3:1-(2-Hydroxy-4-methoxyphenyl)-2-(4-hydroxy-3-methoxy-phenyl)-ethanone (compound 3)

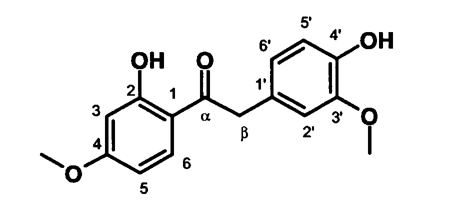

Resorcinol monomethyl ether (0.65 g, 5 mmol.), 4-hydroxy-3-methoxyphenyl-acetic acid (0.93 g, 5 mmol.) and boron trifluoride-ether complex (12.5 ml, 0.1 mol.) were stirred under a nitrogen atmosphere for 3 hours at 20-25°C and then for 2.5 hours at 55-65°C. The cloudy mixture was poured into a sodium acetate solution (12 g/100 ml water) (caution: violent reaction). The reaction mixture was diluted with diethyl ether and washed with dilute hydrochloric acid (15 %), and the organic phase was dried over Na₂SO₄ filtration was carried out, and the organic phase was concentrated to dryness by evaporation *in vacuo.* The dark-red residue (1.6 g) was stirred with a small amount of ethyl acetate to complete the reaction; filtration was carried out, and the residue was washed with a small amount of ethyl acetate.

HPLC-MS (RP-18-Phase, APCl+): *m*/*z* = 285.8 (100 %), 286.3 (13,8 %), 289.3 (2.3 %, [M+H]⁺).

¹H-NMR (400 MHz, CDCl₃, internal standard TMS): δ = 7.76 (1H, d, *J* = 8.7 Hz, H-6), 6.87 (1H, m, *J* = 8.3 Hz, H-5'), 6.77 (1H, s, H-2'), 6.76 (1H, dd, *J* = 8 Hz, *J* = 2 Hz, H-6'), 6.44 (1H, dd, *J* = 8.7 Hz, *J* = 2.5 Hz, H-5), 6.42 (1H, d, *J* = 2.5 Hz, H-3), 5.56 (1H, s, OH), 4.14 (2H, s, H-β), 3.87 (3H, s, -OCH₃'), 3.83 (3H, s, -OCH₃) ppm.

### Application Example 1: Bitter reduction of a solution of bitter substance

In order to quantify the reduction in the bitter impression, the bitterness of a caffeine solution comprising 500 ppm and of a sample comprising 500 ppm of caffeine and a variable amount of the compound of the example was determined by a group of experts (rating 0 [not bitter] to 10 [extremely bitter]). The evaluation was carried out by calculating the reduction (in %) in the bitter impression from the average values of the assessments of the caffeine solution or the solutions containing caffeine and compound of the example. 2,4-Dihydroxybenzoic acid (2,4-DHB) was used as comparison in accordance with US 5,643,941.

| **Substance** | **Bitter substance** | **Bitter (1-10)** | **impression** | **% reduction in the bitter impression** |
|---|---|---|---|---|
| | | **without** | **with** | |
| 100 ppm | 500 ppm caffeine | 5.1 ± 1.0 | 5.0 ± 1.0 | 3% |
| 2,4-DHB | | | | |
| 100 ppm | 500 ppm caffeine | 4.9 ± 1.0 | 3.8 ± 0.9 | 23% |
| (compound 1, Example 1) | | | | |
| 100 ppm | 500 ppm caffeine | 4.7±1.1 4.7 ± 1.1 | 4.3±0.8 4.3 ± 0.8 | 10% |
| (compound 2, Example 2) | | | | |

**Table:** Bitterness (a) of a caffeine solution and (b) of a solution comprising caffeine and a bitter-masking agent; the 95 % confidence intervals are given as error.

### Application Example 2: Enhancement of the sweet impression of a sugar solution

In order to quantify the enhancement of the sweet impression, the sweetness of a sucrose solution comprising 5 % and of a sample comprising 5 % sucrose and an amount of compound 2 was determined by a group of experts (rating 0 [not sweet] to 10 [extremely sweet]). The evaluation was carried out by calculating the reduction (in %) in the sweet impression from the average values of the assessments of the sucrose solution or of the solution comprising sucrose and compound 2.

| **Substance** | | **Sweet (1-10)** | **impresssion** | **% enhancement of the sweet impression** |
|---|---|---|---|---|
| | | **without** | **with** | |
| 100 ppm | 5% sucrose | 5.0 ± 1.2 | 5.9 ± 1.5 | 18% |
| Compound 2 (Example 2) | | | | |

**Table:** Sweetness (a) of a sucrose solution and (b) of a solution comprising sucrose and compound 2; the standard deviations are given as error.

### Application Example 3: Spray-dried preparation in the form of a semi-finished product for flavouring finished products

| **Ingredient** | **Amount in wt.%** |
|---|---|
| Drinking water | 60.8 % |
| Maltodextrin from wheat | 24.3 % |
| Gum arabic | 6.1 % |
| 2-(4-Hydroxy-3-methoxyphenyl)-1-(2,4,6-trihydroxyphenyl)ethanone (compound 1, Example1) | 8.8 % |

The drinking water is placed in a container, and the maltodextrin and the gum arabic are dissolved therein. 2-(4-Hydroxy-3-methoxyphenyl)-1-(2,4,6-trihydroxyphenyl)ethanone (compound 1, Example 1) is then emulsified into the carrier solution using a Turrax. The temperature of the spray solution should not exceed 30°C. The mixture is then spray-dried (desired temperature inlet: 185-195°C, desired temperature outlet: 70-75°C). The spray-dried semi-finished product comprises about 18-22 % of compound 1.

### Application Example 4: Combination with sweeteners

0.5 g of the spray-dried semi-finished product from Application Example 3 is added to 90 g of sucrose and 10 g of tagatose, and the whole is mixed. The product can be used, for example, as a sweetener for coffee or tea.

### Application Example 5: Black tea preparation

| **Ingredient** | **Amount in wt.%** |
|---|---|
| Black tea, Ceylon, leaf | 94.00 % |
| Semi-finished product from Application Example 3, comprising about 18-22 % 2-(4-hydroxy-3-methoxyphenyl)-1-(2,4,6-trihydroxyphenyl)ethanone (compound 1, Example 1) | 6 % |

The tea and the semi-finished product are mixed and packed into tea bags made of filter paper. For use, a tea bag is introduced into 100 to 250 ml of boiling water and allowed to brew for 2 to 5 minutes.

### Application Example 6: Black tea preparation in combination with homoeriodictyol sodium salt

| **Ingredient** | **Amount in wt.%** |
|---|---|
| Black tea, Ceylon, leaf | 94.00 % |
| Semi-finished product from Application Example 3, comprising about 18-22 % 2-(4-hydroxy-3-methoxypheny)-1-(2,4,6-trihydroxypheny)ethanone (compound 1, Example 1) | 3 % |
| Semi-finished product of homoeriodictyol sodium salt according to EP 1258200 B1, spray-dried analogously to Application Example 3 | 3 % |

The tea and the semi-finished products are mixed and packed into tea bags made of filter paper. For use, a tea bag is introduced into 100 to 250 ml of boiling water and allowed to brew for 2 to 5 minutes.

### Application Example 7: Use in a soya drink

The compound 1-(2,4-dihydroxyphenyl)-2-(4-hydroxy-3-methoxyphenyl)ethanone (compound 2, Example 2) was pre-dissolved in ethanol and added to a soya milk from a local supermarket. The mixture was stirred in a glass beaker together with the milk flavour.

| **Ingredient** | **Amount in wt.%** |
|---|---|
| Soya milk (local supermarket) | 99.8 % |
| Milk flavour | 0.1 % |
| 10 % 1-(2,4-dihydroxyphenyl)-2-(4-hydroxy-3-methoxyphenyl)ethanone (compound 2, Example 2) in ethanol | 0.1 % |

### Application Example 8: Use in a soya drink in combination with γ-aminobutyric acid

γ-Aminobutyric acid was pre-dissolved in water and 1-(2,4-dihydroxyphenyl)-2-(4-hydroxy-3-methoxyphenyl)ethanone (compound 2, Example 2) ethanol and added to a soya milk from a local supermarket. The resulting mixture was stirred in a glass beaker together with the milk flavour.

| **Ingredient** | **Amount in wt.%** |
|---|---|
| Soya milk (local supermarket) | 99.7 % |
| Milk flavour | 0.1 % |
| 10 % 1-(2,4-dihydroxyphenyl)-2-(4-hydroxy-3-methoxyphenyl)ethanone (compound 2, Example 2) in ethanol | 0.1% |
| 1 % γ-aminobutyric acid in water | 0.1 % |

### Application Example 9: Use in a chewing gum

| **Part** | **Ingredient** | **Amount in wt.%** |
|---|---|---|
| A | Chewing gum base, Company "Jagum T" | 30.00 |
| B | Sorbitol, powdered | 39.00 |
| | Isomalt^{®} (Palatinit GmbH) | 9.50 |
| | Xylitol | 2.00 |
| | Mannitol | 3.00 |
| | Aspartam^{®} | 0.10 |
| | Acesulfam^{®} K | 0.10 |
| | Emulgum^{®} (Colloides Naturels, Inc.) | 0.30 |
| C | Sorbitol, 70% | 14.00 |
| | Glycerol | 1.00 |
| D | Flavouring, comprising 1 % 1-(2,4-dihydroxyphenyl)-2-(4-hydroxy-3-methoxyphenyl)ethanone (compound 2, Example 2), based on the total weight of the flavouring | 1 |

Parts A to D are mixed and kneaded intensively. The crude mass can be processed, for example, in the form of thin strips to give chewing gum that is ready for consumption.

### Application Example 10: Use in a toothpaste

| **Part** | **Ingredient** | **Amount in wt.%** |
|---|---|---|
| A | Demineralised water | 22.00 |
| | Sorbitol (70%) | 45.00 |
| | Solbrol^{®} M, sodium salt (Bayer AG, p-hydroxybenzoic acid alkyl ester) | 0.15 |
| | Trisodium phosphate | 0.10 |
| | Saccharin, 450 times | 0.20 |
| | Sodium monofluorophosphate | 1.12 |
| | Polyethylene glycol 1500 | 5.00 |
| B | Sident 9 (abrasive silicon dioxide) | 10.00 |
| | Sident 22 S (thickening silicon dioxide) | 8.00 |
| | Sodium carboxymethylcellulose | 0.90 |
| | Titanium dioxide | 0.50 |
| C | Demineralised water | 4.53 |
| | Sodium lauryl sulfate | 1.50 |
| D | Flavouring, comprising 1 % 2-(4-hydroxy-3-methoxyphenyl)-1-(2,4,6-trihydroxyphenyl)-ethanone (compound 1, Example 1), based on the total weight of the flavouring | 1 |

The ingredients of parts A and B are pre-mixed separately, and the parts are then thoroughly stirred together *in vacuo* for 30 minutes at 25-30°C. Part C is pre-mixed and added to A and B; D is added, and the mixture is stirred thoroughly *in vacuo* for 30 minutes at 25 to 30°C. After pressure relief, the toothpaste is finished and can be introduced into containers.

### Application Example 11: Use in a low-fat yoghurt for enhancing sweetness

5 % sugar was stirred into a commercially available natural yoghurt (without additives) having a fat content of 0.1 % (sample 1). 200 ppm of compound 2 (Example 2), which had previously been dissolved in a minimal amount of ethanol, were additionally incorporated into a sample 2. Samples 1 and 2 were presented in coded form, in a different but predetermined order, to 16 testers for tasting. The testers had to note the sweetness impression on a scale of from 0 (not present) to 10 (extreme sweetness). In addition, the testers were to note other taste impressions.

Result: 4 testers found sample 1 sweeter, 10 found sample 2 sweeter, 2 found both to be equally sweet. Sample 1 was rated an average of 4.1, sample 2 4.9 (18 % enhancement of the sweetness impression). The descriptor sticky was noted as a further impression for sample 2. Sample 2 was preferred to sample 1 by the majority of testers.

## Claims

1. Use of hydroxydeoxybenzoins of formula (I) 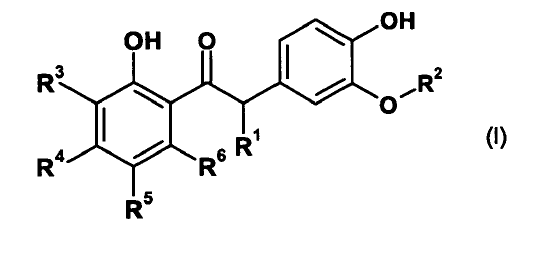 wherein
R¹ and R² independently of one another represent hydrogen or lower alkyl,
R³ R⁴, R⁵ and R⁶ independently of one another represent hydrogen, hydroxy or lower alkoxy,
salts thereof and mixtures thereof to mask or reduce the unpleasant taste impression of a substance that tastes unpleasant and/or to enhance the sweet taste of a substance that tastes sweet.

2. Use according to claim 1, wherein
R¹ represents hydrogen,
R² represents hydrogen or a lower alkyl radical,
R³ and R⁵ represent hydrogen
and
at least one of the radicals R⁴ and R⁶ represents hydroxy or lower alkoxy,
salts thereof and mixtures thereof.

3. Use according to claim 1 or 2, wherein
R¹ represents hydrogen,
R² represents hydrogen or methyl,
R³ and R⁵ represent hydrogen
and
at least one of the radicals R⁴ and R⁶ represents hydroxy or methoxy,
salts thereof and mixtures thereof.

4. Use of
2-(4-hydroxy-3-methoxypheny)-1-(2,4,6-trihydroxyphenyl)ethanone,
1-(2,4-dihydroxyphenyl)-2-(4-hydroxy-3-methoxyphenyl)ethanone or
1-(2-hydroxy-4-methoxyphenyl)-2-(4-hydroxy-3-methoxyphenyl)ethanone,
salts thereof or mixtures thereof
to mask or reduce the unpleasant taste impression of a substance that tastes unpleasant and/or to enhance the sweet taste of a substance that tastes sweet.

5. Use of a salt of a hydroxybenzoin according to any one of the preceding claims, wherein one, more than one or all of the hydroxy groups of the hydroxybenzoic acid amide have been deprotonated and a corresponding amount of counter-cations is present, which counter-cations are selected from the group consisting of: singly positively charged cations of the first main and subsidiary group, ammonium ions, trialkylammonium ions, doubly positively charged cations of the second main and subsidiary group, and triply positively charged cations of the third main and subsidiary group, and mixtures thereof.

6. Use according to any one of the preceding claims in combination with at least one further substance for changing, masking or reducing the unpleasant taste impression of a substance that tastes unpleasant.

7. Use according to any one of the preceding claims in combination with at least one substance that tastes sweet.

8. Use according to any one of the preceding claims in a preparation for nutrition, oral care or enjoyment or in an oral pharmaceutical preparation or in a cosmetic preparation for application in the region of the head.

9. Preparation for nutrition, oral care or enjoyment or preparation for application in the region of the head, comprising from 0.000001 wt.% to 95 wt.%, based on the total weight of the preparation, of a hydroxydeoxybenzoin, a salt or a mixture according to any one of claims 1 to 4.

10. Oral pharmaceutical preparation comprising from 0.000001 wt.% to 10 wt.%, based on the total weight of the preparation, of a hydroxydeoxybenzoin, a salt or a mixture according to any one of claims 1 to 4 and at least one substance that tastes unpleasant.

11. Preparation according to either claim 9 or claim 10, comprising at least one substance that tastes unpleasant, wherein the amount of the substance that tastes unpleasant is sufficient to be perceived as an unpleasant taste in a comparative preparation that does not comprise any hydroxydeoxybenzoin, salt or mixture according to either claim 1 or claim 2, as defined in any one of claims 1 to 4, but otherwise has an identical composition, and the amount of the hydroxydeoxybenzoin, salt or mixture, as defined in any one of claims 1 to 4, in the preparation is sufficient to mask sensorially the unpleasant taste impression of the substance that tastes unpleasant or to reduce it in comparison with the comparative preparation.

12. Preparation according to any one of claims 9 to 11, **characterised in that** it is in the form of a semi-finished product, in the form of a fragrance, flavouring or taste-imparting composition or in the form of a spice mixture.

13. Preparation according to any one of claims 9 to 12, further comprising at least one further substance for changing, masking or reducing the unpleasant taste impression of a substance that tastes unpleasant.

## Patentansprüche

1. Verwendung von Hydroxydeoxybenzoinen der Formel (I) 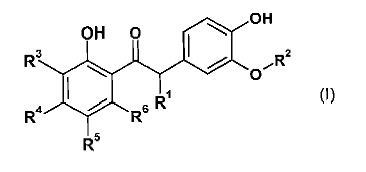 wobei
R¹ und R² unabhängig voneinander Wasserstoff oder Niederalkyl bedeuten,
R³, R⁴, R⁵ und R⁶ unabhängig voneinander Wasserstoff, Hydroxy oder Niederalkoxy bedeuten,
deren Salzen und deren Gemischen zur Maskierung oder Verminderung des unangenehmen Geschmackseindrucks eines unangenehm schmeckenden Stoffes und/oder zur Verstärkung des süßen Geschmacks eines süß schmeckenden Stoffes.

2. Verwendung nach Anspruch 1, wobei
R¹ Wasserstoff bedeutet,
R² Wasserstoff oder Niederalkylrest bedeutet,
R³ und R⁵ Wasserstoff bedeuten
und
mindestens einer der Reste R⁴ und R⁶ Hydroxy oder Niederalkoxy bedeutet, deren Salzen und deren Gemischen.

3. Verwendung nach Anspruch 1 oder 2, wobei
R¹ Wasserstoff bedeutet,
R² Wasserstoff oder Methyl bedeutet,
R³ und R⁵ Wasserstoff bedeuten
und
mindestens einer der Reste R⁴ und R⁶ Hydroxy oder Methoxy bedeutet,
deren Salzen und deren Gemischen.

4. Verwendung von
2-(4-Hydroxy-3-methoxyphenyl)-1-(2,4,6-trihydroxyphenyl)ethanon,
1-(2,4-Dihydroxyphenyl)-2-(4-hydroxy-3-methoxyphenyl)ethanon
oder
1-(2-Hydroxy-4-methoxyphenyl)-2-(4-hydroxy-3-methoxyphenyl)ethanon,
deren Salzen oder deren Gemischen
zur Maskierung oder Verminderung des unangenehmen Geschmackseindrucks eines unangenehm schmeckenden Stoffes und/oder zur Verstärkung des süßen Geschmacks eines süß schmeckenden Stoffes.

5. Verwendung eines Salzes eines Hydroxybenzoins gemäß einem der vorangehenden Ansprüche, wobei eine, mehrere oder sämtliche HydroxyGruppen des Hydroxybenzoesäureamids deprotoniert sind und eine entsprechende Menge von Gegenkationen vorliegt, die ausgewählt sind aus der Gruppe bestehend aus: einfach positiv geladene Kationen der ersten Haupt- und Nebengruppe, Ammoniumionen, Trialkylammoniumionen, zweifach positiv geladene Kationen der zweiten Haupt- und Nebengruppe sowie dreifach positiv geladene Kationen der dritten Haupt- und Nebengruppe und deren Mischungen.

6. Verwendung nach einem der vorangehenden Ansprüche in Kombination mit zumindest einer weiteren Substanz zum Verändern, Maskieren oder Vermindern des unangenehmen Geschmackseindrucks eines unangenehm schmeckenden Stoffes.

7. Verwendung nach einem der vorangehenden Ansprüche in Kombination mit zumindest einem süß schmeckenden Stoff.

8. Verwendung nach einem der vorangehenden Ansprüche in einer der Ernährung, der Mundpflege oder dem Genuss dienenden oder oralen pharmazeutischen Zubereitung oder kosmetischen Zubereitung zur Applikation im Bereich des Kopfes.

9. Der Ernährung, der Mundpflege oder dem Genuss dienende Zubereitung oder Zubereitung zur Applikation im Bereich des Kopfes, umfassend 0.000001 Gew.-% bis 95 Gew.%, bezogen auf das Gesamtgewicht der Zubereitung, eines Hydroxydeoxybenzoins, eines Salzes oder eines Gemisches nach einem der Ansprüche 1 bis 4.

10. Orale pharmazeutische Zubereitung, umfassend 0,000001 Gew.-% bis 10 Gew.%, bezogen auf das Gesamtgewicht der Zubereitung, eines Hydroxydeoxybenzoins, eines Salzes oder eines Gemisches nach einem der Ansprüche 1 bis 4 und mindestens einen unangenehm schmeckenden Stoff.

11. Zubereitung nach einem der Ansprüche 9 oder 10, umfassend zumindest einen unangenehm schmeckenden Stoff, wobei die Menge des unangenehm schmeckenden Stoffes ausreicht, um in einer Vergleichszubereitung, die kein Hydroxydeoxybenzoin, Salz oder Gemisch nach einem der Ansprüche 1 bis 2 umfasst, wie es in einem der Ansprüche 1 bis 4 definiert ist, aber ansonsten identisch zusammengesetzt ist, als unangenehmer Geschmack wahrgenommen zu werden, und die Menge des Hydroxydeoxybenzoins, des Salzes oder des Gemisches, wie es in einem der Ansprüche 1 bis 4 definiert ist, in der Zubereitung ausreicht, um den unangenehmen Geschmackseindruck des unangenehm schmeckenden Stoffes sensorisch zu maskieren oder im Vergleich mit der Vergleichszubereitung zu vermindern.

12. Zubereitung nach einem der Ansprüche 9 bis 11, **dadurch gekennzeichnet, dass** sie als Halbfertigware, als Riech-, Aroma- oder Geschmacksstoffkomposition oder als Würzmischung vorliegt.

13. Zubereitung nach einem der Ansprüche 9 bis 12, weiter umfassend zumindest eine weitere Substanz zum Verändern, Maskieren oder Vermindern des unangenehmen Geschmackseindrucks eines unangenehm schmeckenden Stoffes.

## Revendications

1. Utilisation d'hydroxydésoxybenzoïnes de la formule (I) 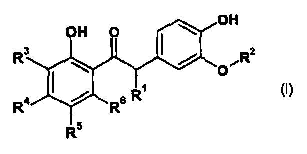 où
R¹ et R² représentent indépendamment l'un de l'autre l'hydrogène ou un alkyle inférieur,
R³, R⁴, R⁵ et R⁶ représentent indépendamment l'un de l'autre l'hydrogène, un hydroxy ou un alcoxy inférieur,
leurs sels et mélanges pour masquer ou réduire le goût désagréable d'une substance qui a un goût désagréable et/ou pour améliorer le goût sucré d'une substance qui a un goût sucré.

2. Utilisation selon la revendication 1, dans lequel
R¹ représente l'hydrogène,
R² représente l'hydrogène ou un radical alkyle inférieur,
R³ et R⁵ représentent l'hydrogène
et
au moins l'un des radicaux R⁴ et R⁶ représente un hydroxy ou un alcoxy inférieur, leurs sels et mélanges.

3. Utilisation selon la revendication 1 ou 2, dans laquelle
R¹ représente l'hydrogène,
R² représente l'hydrogène ou un méthyle,
R³ et R⁵ représentent l'hydrogène
et
au moins l'un des radicaux R⁴ et R⁶ représente un hydroxy ou un méthoxy inférieur, leurs sels et mélanges.

4. Utilisation de
2-(4-hydroxy-3-méthoxyphényl)-1-(2,4,6-trihydroxyphényl)éthanone,
1-(2,4-dihydroxyphényl)-2-(4-hydroxy-3-méthoxyphényl)éthanone,
ou
1-(2-hydroxy-4-méthoxyphényl)-2-(4-hydroxy-3-méthoxyphényl)éthanone,
leurs sels et mélanges
pour masquer ou réduire le goût désagréable d'une substance qui a un goût désagréable et/ou pour améliorer le goût sucré d'une substance qui a un goût sucré.

5. Utilisation d'un sel d'un hydroxybenzoïne selon l'une quelconque des revendications précédentes, dans laquelle un groupe hydroxy, plus d'un groupe hydroxy ou tous les groupes hydroxy de l'amide de l'acide hydroxybenzoïque ont été déprotonés et une quantité correspondante, de contre-cations est présente, lesquels contre-cations sont sélectionnés dans le groupe comprenant des cations simplement chargés positivement du premier groupe principal et subsidiaire, des ions ammonium, des ions trialkylammonium, des cations doublement chargés positivement du deuxième groupe principal et subsidiaire, et des cations triplement chargés positivement du troisième groupe principal et subsidiaire, et leurs mélanges.

6. Utilisation selon l'une quelconque des revendications précédentes en combinaison avec au moins une autre substance pour modifier, masquer ou réduire le goût désagréable d'une substance qui a un goût désagréable.

7. Utilisation selon l'une quelconque des revendications précédentes en combinaison avec au moins une substance qui a un goût sucré.

8. Utilisation selon l'une quelconque des revendications précédentes dans une préparation pour aliment, pour soin buccal ou pour le plaisir ou dans une préparation pharmaceutique à usage oral ou dans une préparation cosmétique destinée à être appliquée dans la région de la tête.

9. Préparation pour aliment, pour soin buccal ou pour le plaisir ou préparation destinée à être appliquée dans la région de la tête, comprenant de 0,000001% en poids à 95% en poids, rapportés au poids total de la préparation, d'un hydroxydésoxybenzoïne, d'un sel ou d'un mélange selon l'une quelconque des revendications 1 à 4.

10. Préparation pharmaceutique à usage oral comprenant de 0,000001% en poids à 10% en poids, rapportés au poids total de la préparation, d'un hydroxydésoxybenzoïne, d'un sel ou d'un mélange selon l'une quelconque des revendications 1 à 4 et au moins une substance qui a un goût désagréable.

11. Préparation selon la revendication 9 ou la revendication 10, comprenant au moins une substance qui a un goût désagréable, dans laquelle la quantité de substance qui a un goût désagréable est suffisante pour être perçue comme un goût désagréable dans une préparation comparative qui ne comprend pas d'hydroxydésoxybenzoïne, de sel ou de mélange selon la revendication 1 ou la revendication 2, comme défini dans l'une quelconque des revendications 1 à 4, mais qui a par ailleurs une composition identique, et la quantité d'hydroxydésoxybenzoïne, de sel et de mélange, comme défini dans l'une quelconque des revendications 1 à 4, dans la préparation est suffisante pour masquer du point de vue sensoriel le goût désagréable de la substance qui a un goût désagréable ou pour le réduire par rapport à la préparation comparative.

12. Préparation selon l'une quelconque des revendications 9 à 11, **caractérisée en ce qu'**elle se présente sous la forme d'un produit semi-fini, sous la forme d'une composition communiquant un parfum, un arôme ou un goût ou sous la forme d'un mélange d'épices.

13. Préparation selon l'une quelconque des revendications 9 à 12, comprenant en outre au moins une autre substance pour modifier, masquer ou réduire le goût désagréable d'une substance qui a un goût désagréable.
